# EUROPEAN PATENT APPLICATION

(11) **EP 4 331 508 A1**
(43) Date of publication of application: **06.03.2024**
(21) Application number: 22794616.7
(22) Date of filing: 15.04.2022
(51) Int. Cl.: A61B 17/22, A61M 25/09, A61M 25/00

(54) **MEDICAL DEVICE**

(30) Priority: 26.04.2021 CN 202120869452 U; 17.08.2021 CN 202110942300
(71) Applicant: Shanghai Shenqi Medical Technology Co., Ltd., Shanghai 201201 (CN)
(72) Inventor: YANG, Kun, Shanghai 201201 (CN); ZHOU, Guolei, Shanghai 201201 (CN); WANG, Sen, Shanghai 201201 (CN); HUANG, Zhongrong, Shanghai 201201 (CN); DAI, Zhihao, Shanghai 201201 (CN)
(74) Representative: Cabinet Laurent & Charras
(86) International application number: PCT/CN2022/086963
(87) International publication number: WO 2022/228145

(57) **Abstract**

A medical device, the medical device comprising a joint (1), a catheter (2), and a guide wire (3). The medical device further comprises: an elastic part (4) sleeved on the other end of the guide wire (3); and a protruding part (5) sleeved on the outer circumference of the guide wire (3). The protruding part (5) is connected between the elastic part (4) and the catheter (2); the diameter of the protruding part (5) is larger than the diameters of the catheter (2) and the elastic part (4). When the catheter (2) is drawn inside a blood vessel, the protruding part (5) can not only prevent damage to the medical device, but also effectively lower the risk in catheter-directed thrombolysis, and can also break and clean thrombi in advance, so as to increase the contact area of a liquid medicine during medication, thereby accelerating absorption of the liquid medicine and improving treatment effect.

## Description

### TECHNICAL FIELD

The present disclosure relates to the technical field of medical instruments, and particularly to a medical device.

### BACKGROUND

Peripheral vascular thrombus-obstructive disease is categorized into venous thromboembolism and arterial thromboembolism. Current conventional thrombosis treatments include anticoagulation, surgical treatment and catheter-directed thrombolysis. Among them, the mechanism of catheter-directed thrombolysis is to directly perfuse high concentration thrombolytic medicine around the thrombus through a thrombolytic catheter to dissolve thrombus and achieve therapeutic purpose. Compared with traditional catheter thrombectomy, catheter-directed thrombolysis not only does not require general anesthesia and has little surgical trauma, but can also avoid repeated dragging and pulling of the catheter inside the blood vessel, which reduces a damage to the blood vessel, and is therefore an ideal method of thrombosis treatment.

In the process of catheter-directed thrombolysis, when perfusing thrombolytic medicine liquid into human body by a thrombolytic catheter, the medicine liquid flows out from the liquid outlet hole of the catheter to flush the affected area of the thrombus, such that achieves the purpose of ultimately dissolving the thrombus. In conventional arts, the thrombolytic catheter includes a joint, a guiding wire, a catheter and a blocking member. The catheter is connected to the joint at one end and connected to the blocking member at the other end. The guiding wire is provided inside the catheter, and is connected to the joint at one end and extends out of the catheter at the other end. And the blocking member is sleeved on the guiding wire extending out of the catheter, and one end of the blocking member can be extended into the catheter, such that the blocking member and the catheter can form a sealing structure, and prevent the medicinal liquid from directly flowing out from a central hole at an end of the catheter, and meanwhile, the blocking member can avoid injury of the blood vessel by a tip of the guiding wire. A difference between a diameter of the blocking member and a diameter of the catheter is very small, but due to the fact that a location of the thrombosis is not fixed, i.e., the catheter needs to pass through a variety of curved blood vessel passages within the human body, and the blocking member may be friction with an inner wall of the blood vessel and thus be damaged when the catheter is pulled in the blood vessel, which leads to a damage of the thrombolytic catheter, and at the same time, the blood vessel of the human body may be damaged, increasing the risk of catheter-directed thrombolysis. Furthermore, when the catheter reaches the location of serious embolism in the blood vessel and there is a large-area thrombus block in the blood vessel, the medicinal liquid flowing out from the liquid outlet hole of the catheter cannot dissolve the thrombus sufficiently, which greatly reduces a therapeutic effect.

Accordingly, the aforementioned problems need to be solved urgently.

### SUMMARY

Accordingly, a medical device is disclosed in the present disclosure, the medical device can not only prevent being damaged when the catheter is drawn out, so as to effectively reduce the risk of catheter-directed thrombolysis, but also can dissolve the thrombus sufficiently and improve the therapeutic effect.

A medical device includes a joint, a catheter and a guiding wire, an end of the catheter being connected to the joint, the guiding wire being provided inside the catheter, one end of the guiding wire being connected to the joint, and the other end of the guiding wire extends out of the catheter. The medical device further includes:
an elastic portion sleeved on the other end of the guiding wire; and
a protruding portion sleeved on a periphery of the guiding wire, the protruding portion is connected between the elastic portion and the catheter, and a diameter of the protruding portion is greater than diameters of the catheter and the elastic portion.

In one of embodiments, the protruding portion includes two protruding members, the protruding members are shaped as a truncated cone, lower bottom surfaces of the two protruding members are attached, and upper bottom surfaces of the two protruding members are connected to the catheter and the elastic portion, respectively.

In one of embodiments, the two protruding members are symmetrically arranged.

In one of embodiments, a diameter of the guiding wire gradually decreases from the joint towards the elastic portion.

In one of embodiments, the diameter of the catheter gradually decreases from the joint towards the protruding portion.

In one of embodiments, a wall of the catheter at the other end is provided with a plurality of liquid outlet holes, and an opening of the catheter at the other end is sealed.

In one of embodiments, the catheter includes:
a first catheter portion connected to the j oint; and
a second catheter portion connected between the first catheter portion and the protruding portion, a hardness of the second catheter portion is greater than a hardness of the first catheter portion, and the plurality of liquid outlet holes are located on the second catheter portion.

In one of embodiments, the elastic portion includes at least one spring.

In one of embodiments, the elastic portion comprises a second spring and a third spring, the second spring is sleeved on a periphery of the third spring, the end of the guiding wire extending out of the catheter is coplanar with an end of the third spring, and the end of the third spring is coplanar with an end of the second spring.

In one of embodiments, a fatigue strength of the second spring is greater than a fatigue strength of the third spring.

In one of embodiments, a developing capacity of the third spring is greater than a developing capacity of the second spring.

In one of embodiments, an end of the elastic portion away from the catheter is provided with a hemispherical terminal.

According to the above technical solution, a medical device is disclosed in the present disclosure. In the present disclosure, an opening of the catheter at an end away from the joint is sealed, so as to prevent the medicine liquid injected into the catheter from flowing out from the opening of the catheter at the end away from the joint. Meanwhile, an elastic portion is provided in the present disclosure, which can prevent the tip of the guiding wire extending out of the catheter from injuring the blood vessel. In addition, due to the elasticity of the elastic portion itself, the influence of the guiding wire moving in various curved blood vessels in human body is greatly reduced. The medical device is further provided with a protruding portion, when the catheter is pulled in the blood vessel, the protruding portion can provide appropriate space for the elastic portion and reduce the contact between the elastic portion and the inner wall of the blood vessel, which not only can avoid the damage to the medical device when the catheter is drawn outward, but also can effectively reduce the risk of catheter-directed thrombolysis. When the medical device moves towards a location in the blood vessel with severe embolism, the protruding portion adjacent to the elastic portion can mechanically crush and unblock the thrombus in advance, thereby increasing the contact area of the medicine liquid and accelerating the absorption rate of the medicine liquid during subsequent medicine administration, thus the therapeutic effect is improved.

### BRIEF DESCRIPTION OF THE DRAWINGS

To illustrate the technical solutions according to the embodiments of the present invention or in the prior art more clearly, the accompanying drawings for describing the embodiments or the prior art are introduced briefly in the following. Apparently, the accompanying drawings in the following description are only some embodiments of the present invention, and persons of ordinary skill in the art can derive other drawings from the accompanying drawings without creative efforts.
FIG. 1 is a schematic view of a medical device according to an embodiment of the present disclosure.
FIG. 2 is a partial enlarged view of portion A in FIG. 1;
FIG. 3 is a partial enlarged view of portion B in FIG. 1.

In drawings:
1: joint; 11: connecting tube;
2: catheter; 21: first catheter portion; 22: second catheter portion; 221: liquid outlet hole;
3: guiding wire;
4: elastic portion; 41: terminal;
5: protruding portion; 51: protruding member.

### DETAILED DESCRIPTION OF THE EMBODIMENTS

The present disclosure will be described in detail hereinafter in combination with the accompanying drawings and embodiments. It should be understood that the specific embodiments described herein are only used to explain the present disclosure, and shall not be considered as a limitation to the present disclosure. It should also be noted that, for the sake of description, the accompanying drawings show only part of the structure related to the present disclosure and not all of it.

In the description of the present disclosure, the terms "connecting together," "connecting," "fixing" and the like should be understood broadly. For example, it could be understood as a fixed connection, a detachable connection, or an integrated connection; it could be understood as a mechanical connection or an electrical connection; it could be understood as a direct connection or an indirect connection by an intermediate medium; and it could be understood as an internal communication between two elements or an interaction between two elements. For those skill in the art, the specific meaning of the aforementioned terms in the present disclosure can be understood according to specific situations.

In the present disclosure, unless otherwise expressly defined, a first feature being "above" or "below" a second feature may include the first feature is directly contact with the second feature, or may include the first feature is not directly contact with the second feature but through additional features between them. Furthermore, the first feature being "over", "above" and "on top of' the second feature may include the first feature is directly above or obliquely above the second feature, or only means that the level of the first feature is higher than that of the second feature in a horizonal direction. The first feature being "below", "underneath" or "under" the second feature may include the first feature is directly underneath or obliquely underneath the second feature, or only means that the level of the first feature is lower than that of the second feature.

In the description of embodiments, orientation or position relationships of the terms "on", "under", "right" and the like are orientation or position relationships shown based on the accompany drawings, and are merely for convenience of description and simplifying operation, rather than indicating or implying that the indicated medical device or element must have a particular orientation or being constructed and operated in a particular orientation, and are therefore not to be construed as limitation of the present disclosure. In addition, the terms "first", "second" are merely for distinction of description and have no special meaning.

In conventional arts, a medical device includes a joint, a guiding wire, a catheter and a blocking member. The joint can be connected to an external instrument, which can inject a thrombolytic solution into the catheter. The guiding wire can reinforce the hardness of the catheter, it can not only guide and support the catheter to go through soft tissues such as subcutaneous tissue and vessel walls, and enter the blood vessel through the puncture hole, but also can guide the catheter to go through tortuous and sclerotic blood vessels, and selectively enter an examined branch of blood vessel. The blocking member is sleeved on the guiding wire extending out of the catheter, so as to prevent the tip of the guiding wire from injuring the blood vessel. One end of the blocking member can extend into the catheter, such that the blocking member and the catheter can form a sealing structure, thus preventing the medicinal liquid from directly flowing out from a central hole at an end of the catheter, ensuring the therapeutic effect. However, since there is little difference between a diameter of the blocking member and a diameter of the catheter, when the catheter is drawn outwards, the blocking member may be damaged due to a resistance caused by blood flow and a frictional resistance.

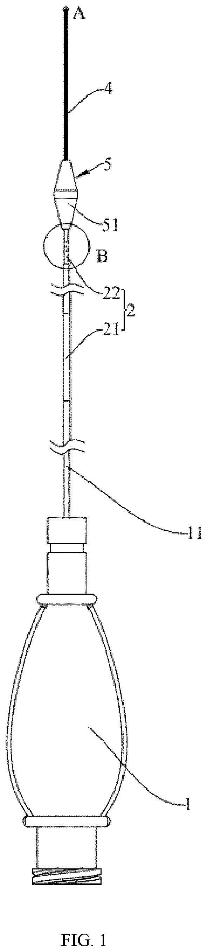

To solve the above problems, a medical device is provided in the present embodiment. Referring to FIG. 1 to FIG. 3, the medical device includes a joint 1, a catheter 2, and a guiding wire 3. One end of the catheter 2 is connected to the joint 1, a wall of the other end of the catheter 2 is provided with a plurality of liquid outlet holes 221, and medicine liquid injected into the catheter 2 can flow out from the plurality of liquid outlet holes 221. The guiding wire 3 is provided inside the catheter 2, one end of the guiding wire 3 is connected to the joint 1, the other end of the guiding wire 3 extends out of the catheter 2, and an opening of the other end of the catheter 2 is sealed. The medical device further includes an elastic portion 4 and a protruding portion 5. The elastic portion 4 is sleeved on the other end of the guiding wire 3. The protruding portion 5 is sleeved on a periphery of the guiding wire 3. The protruding portion 5 is connected between the elastic portion 4 and the catheter 2, and a diameter of the protruding portion 5 is greater than a diameter of the catheter 2 and a diameter of the elastic portion 4.

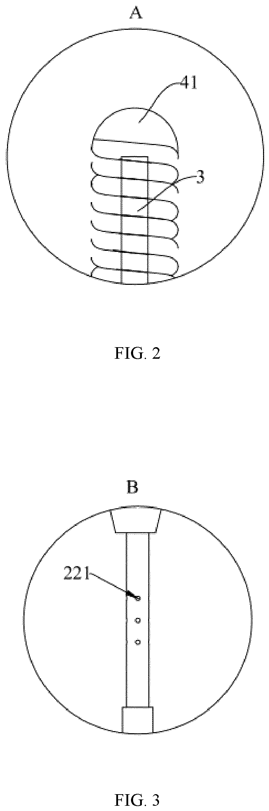

As shown in FIG. 1 and FIG. 2, an opening of the catheter 2 at an end away from the joint 1 is sealed, so as to prevent the medicine liquid injected into the catheter 2 from flowing out from the opening of the catheter 2 at the end away from the joint 1. Meanwhile, the elastic portion 4 is provided in the present embodiment, which can prevent the tip of the guiding wire 3 extending out of the catheter 2 from injuring the blood vessel. In addition, due to the elasticity of the elastic portion 4 itself, the influence of the guiding wire 3 moving in various curved blood vessels in human body is greatly reduced. The medical device is further provided with the protruding portion 5, when the catheter is pulled in the blood vessel, the protruding portion 5 can provide appropriate space for the elastic portion 4 and reduce the contact between the elastic portion 4 and the inner wall of the blood vessel, which not only can avoid the damage to the medical device when the catheter 2 is drawn outward, but also can effectively reduce the risk of catheter-directed thrombolysis. When the medical device moves towards a location in the blood vessel with severe embolism, the protruding portion 5 adjacent to the elastic portion 4 can mechanically crush and unblock the thrombus in advance, thereby increasing the contact area of the medicine liquid and accelerating the absorption rate of the medicine liquid during subsequent medicine administration, thus the therapeutic effect is improved.

Combined with the aforementioned description, since the protruding portion 5 adjacent to the elastic portion 4 can mechanically crush and unblock the thrombus in advance, in addition to being used as a thrombolytic catheter for thrombolysis, the medical device in the present embodiment can also be used as an unblocking catheter alone. When the aforementioned medical device is used merely as the unblocking catheter, the protruding portion 5 can mechanically crush and unblock the thrombus in the blood vessel, such that a pathway for other medical devices to pass through is formed in the blood vessel. When the aforementioned medical device is used as the thrombolytic catheter, the medicine liquid injected into the catheter 2 can effectively dissolve the crushed thrombus. In other words, when the aforementioned medical device is used as the thrombolytic catheter in an interventional surgery, the interventional surgery does not need to unblock the blood vessel using special unblocking catheter, which not only greatly reduces the cost of treatment for the patients, but also improves operability and efficiency of the interventional surgery, and reduces the difficulty of operation of the interventional surgery.

Specifically, in the present embodiment, the opening of the end of the catheter 2 away from the joint 1 is sealed from the guiding wire 3, which can prevent the medicine liquid injected into the catheter 2 from flowing out from the opening of the end away from the joint 1 of the catheter 2, and thereby ensuring the therapeutic effect. No blocking member is required in the present embodiment, but in order to prevent the tip of the guiding wire 3 extending out of the catheter 2 from injuring the blood vessel, and in order to minimize the influence of the guiding wire 3 moving in various curved blood vessels in human body, the elastic portion 4 is adaptively provided in the present embodiment. The elastic portion 4 can wrap the guiding wire 3, and due to its elasticity, the elastic portion 4 can prevent the tip of the guiding wire 3 from injuring the blood vessel without interfering with the movement of the guiding wire 3. However, the smooth catheter 2 cannot ensure that the elastic portion 4 will not be damaged due to the frictional resistance against the vessel wall when the catheter 2 is pulled in the blood vessel. In order to avoid the damage to the elastic portion 4, in the present embodiment, the medical device is further provided with the protruding portion 5. Both ends of the protruding portion 5 are connected to the elastic portion 4 and the catheter 2, respectively. In other words, the guiding wire can extend through the protruding portion 5, and the diameter of the protruding portion 5 is greater than the diameters of the catheter 2 and the elastic portion 4. When the catheter is pulled in the blood vessel, the protruding portion 5 can provide appropriate space for the elastic portion 4, which not only reduces the contact between the elastic portion 4 and the inner wall of the blood vessel, avoids the damage to the elastic portion 4 by pushing and pulling, but also avoids injury to blood vessels of human body, thus reducing the risk of catheter-directed thrombolysis. Furthermore, when the medical device moves towards the thrombosis affected location and enters the location in the blood vessel with severe embolism, the protruding portion 5 adjacent to the elastic portion 4 can mechanically crush and unblock a thrombus block with large volume in advance due to its large diameter, the thrombus block with large volume is crushed into the thrombus block with smaller volume, thereby increasing the contact area of the medicine liquid and thrombus block, and accelerating the absorption rate of the medicine liquid during subsequent medicine administration, thus improving the therapeutic effect.

It should be understood that, in the conventional art, the guiding wire and the catheter of the thrombolytic catheter are independent structures. When the catheter-directed thrombolysis is performed, the guiding wire moves in advance within the blood vessel by a certain distance, then the catheter follows the pathway established by the guiding wire, and passes through the blood vessel and reaches the lesion location through the coordinated operation of the guiding wire and the catheter. In the present embodiment, the guiding wire and the catheter are fixedly connected, the catheter can move along with the guiding wire, such that the medical staff can simultaneously place the guiding wire and catheter into the lesion location through the blood vessel at one time, which simplifies surgery steps, shortens surgery time, reduces surgery difficulty, and improves surgery success rate.

In the present embodiment, the aforementioned joint 1 is a Luer taper 1. Since the Luer taper 1 is a structure known to those skilled in the art, the structure and working principle of the Luer taper 1 will not be repeated herein.

It should be understood that, the sealing between the opening of the end of the catheter 2 away from the joint 1 and the guiding wire 3 is implemented by dispensing glue. Of course, in other optional embodiments, other means may also be used to seal between the opening of the end of the catheter 2 away from the joint 1 and the guiding wire 3, which is not specifically limited herein.

In order to easily control the movement of the guiding wire 3 in the blood vessel, the guiding wire 3 away from the joint 1 needs to be sufficiently flexible, while the guiding wire 3 adjacent to the joint 1 needs to be sufficiently rigid. Therefore, in the present embodiment, the diameter of the guiding wire 3 gradually decreases from the joint 1 towards the elastic portion 4, and the diameter of the catheter 2 gradually decreases from the joint 1 towards the protruding portion 5. The thinner guiding wire 3 can meet the flexibility required for its movement in the curved blood vessel, and the thicker guiding wire 3 can meet the rigidity required to control its movement. At the same time, since the diameter of the blood vessels in the body gradually decreases from the main circuit to the branches to the endings, the catheter 2 with decreasing diameter can adapt to the changes in the diameter of the blood vessels.

Referring to FIG. 1, the protruding portion 5 includes two protruding members 51, the two protruding members 51 are symmetrically arranged and are shaped as the truncated cone. Lower bottom surfaces of the two protruding members 51 are attached, and upper bottom surfaces of the two protruding members 51 are connected to the catheter 4 and the elastic portion 4, respectively. Pathways are formed between the upper bottom surfaces and lower bottom surfaces of the two protruding members 51, and the pathways of the two protruding members 51 are communicated. The end of the catheter 2 away from the joint 1 can extend into one pathway of the protruding member 51, meanwhile the guiding wire 3 can extend out from the other pathway of the protruding member 51. The end of the elastic portion 4 adjacent to the protruding portion 5 extends into the other pathway of the protruding member 51. The connection position of the catheter 2 and the protruding portion 5, and the connection position of the elastic portion 4 and the protruding portion 5 are welded. Since both the two protruding members 51 are shaped as the truncated cone, i.e., the side surfaces of the two protruding members 51 are inclined surfaces, when the medical device enters the blood vessel and moves towards the affected location of thrombus or the catheter 2 is drawn outwards, not only the contact between the elastic portion 4 and the inner wall of the blood vessel can be reduced, but also the resistance of the blood can be effectively reduced. In addition, when the medical device moves towards the affected location of thrombus and enters the location in the blood vessel with severe embolism, the protruding portion 5 with the inclined surface is beneficial to crush the thrombus block with a larger volume into thrombus block with a smaller volume, and can mechanically crush and unblock the thrombus with the larger volume, thereby increasing the contact area of the medicine liquid and thrombus block, accelerating the absorption rate of the medicine liquid during subsequent medicine administration, and further improving the therapeutic effect.

It should be understood that, in order to facilitate manufacturing, in the present embodiment, the two protruding members 51 are integrally formed.

Furthermore, the protruding portion 5 is made of soft material. In the present embodiment, the protruding portion 5 is made of Pebax material, which can avoid injury to the blood vessel of human body.

Since the affected location of thrombus is not fixed, after entering the human body, the catheter 2 is required to pass through various curved blood vessels and finally enter the affected location of thrombus. It is required to prevent the catheter 2 from damaging the blood vessel during its movement. Therefore, the catheter 2 in the conventional art is usually made of soft material, and the liquid outlet hole 221 is formed on the catheter 2 of soft material by laser-cutting. As the soft material is soft and easy to be deformed, the processability is poor, hence the precision of the cut hole is low, and is prone to burrs, deformation and other problems. The low precision of the liquid outlet hole 221 may lead to a large deviation between the theoretical value and the actual value of the liquid outlet rate, thus affecting the calculation of the actual amount of medicine liquid, that is, affecting the medical staffs control of the amount of medication. In addition, the liquid outlet hole 221 on the catheter 2 made of soft material may be deformed by force and lead to the decreasing of the hole size or clogging of the hole, which results in the reduction of the outflow amount of the medicine liquid or even cannot be outflowed. Accordingly, referring to FIG. 1 and FIG. 3, the catheter 2 in the present embodiment includes the first catheter portion 21 and the second catheter portion 22. The first catheter portion 21 is connected to the joint 1, and the first catheter portion 21 is made of soft material. The second catheter portion 22 is connected between the first catheter portion 21 and the protruding portion 5. A plurality of liquid outlet holes 221 are located on the second catheter portion 22, and the second catheter portion 22 is made of metal material. Metal material has higher hardness, thus the hardness of the second catheter portion 22 is greater than the hardness of the first catheter portion 21, which makes it easy to process the plurality of liquid outlet holes 221, and the precision of the processed holes is high. In addition, it is ensured by the second catheter portion 22 made of metal material that the medicine is released stably through the plurality of liquid outlet holes 221 in all four directions of a circle of the catheter 2. Furthermore, the metal has developing capacity, which enables the accurate location of the liquid outlet holes 221 in the clinical surgery, while on the contrary, the soft material does not have developing capacity and the liquid outlet holes 221 cannot be located. Moreover, in the present embodiment, a length of the first catheter portion 21 is much greater than a length of the second catheter portion 22, which greatly reduces the influence on the movement of the catheter 2.

It should be understood that the second catheter portion 22 may be made of high-density metal such as gold, platinum, and tungsten, the aforementioned metals are more capable of developing, which is not specifically limited herein.

Furthermore, by processing the liquid outlet hole 221 on the second catheter portion 22 made of metal material, not only the processing precision of the liquid outlet hole 221 is ensured, but also the processing of the liquid outlet hole 221 with a very small size is enabled. The smaller liquid outlet hole 221 is beneficial to the medical staff to more accurately control the medication amount. In the present embodiment, the cross-sectional area of the liquid outlet hole 221 is specifically 0.03 square millimeter. Of course, in other optional embodiments, the cross-sectional area of the liquid outlet hole 221 may also be other values, which is not specifically limited herein.

As shown in FIG. 1, the joint 1 includes a connecting tube 11. The first catheter portion 21 is connected to the joint 1 through the connecting tube 11, one end of the guiding wire 3 extends into the connecting tube 11, and a hardness of the connecting tube 11 is greater than the hardness of the first catheter portion 21. In the present embodiment, the catheter 2 is connected to the Luer taper 1 through the connecting tube 11, which can prevent bending at the connection position of the catheter 2 and the Luer taper 1.

It should be understood that one end of the guiding wire 3 provided inside the catheter 2 adjacent to the Luer taper 1 can extend into and be welded in the connecting tube 11.

In the present embodiment, the elastic portion 4 includes a spring, i.e., the elastic portion 4 is a first spring, which can prevent the tip of the guiding wire 3 extending out of the catheter 2 from injuring the blood vessel. In addition, due to the elasticity of the elastic portion 4 itself, the influence of the guiding wire 3 moving in various curved blood vessels in human body is greatly reduced. The first spring is made of metal material, due to the developing capacity of metal material, medical staff can monitor the position of the guiding wire 3 in real time. Meanwhile, the manufacturing cost of medical devices is reduced.

As shown in FIG. 2, an end of the elastic portion 4 away from the catheter 2 is provided with a hemispherical terminal 41. Welding the hemispherical terminal 41 at the end of the elastic portion 4 away from the catheter 2 can avoid injury to the blood vessel during movement of the elastic portion 4.

### Embodiment 2

Comparing with Embodiment 1, the difference of the present embodiment lies in that the elastic portion 4 includes a second spring and a third spring. The second spring is sleeved on a periphery of the third spring. The end of the guiding wire 3 extending out of the catheter 2 is coplanar with an end of the second spring, and an end of the third spring is coplanar with the end of the second spring. The elastic portion 4 is made of metal material, due to the developing capacity of metal material, the medical staff can monitor the position of the guiding wire 3 in real time.

Furthermore, the second spring is made of high strength metal material and the third spring is made of high-density metal material. A fatigue strength of the second spring is greater than a fatigue strength of the third spring, and a developing capacity of the third spring is greater than a developing capacity of the second spring. As the second spring has higher fatigue strength, it can provide higher elasticity and is not easy to be damaged by pulling and bending. The second spring is optionally made of stainless steel. In order to ensure that medical staff can monitor the position of the guiding wire 3 accurately, the third spring is provided within the second spring, and the third spring is optionally made of gold, platinum, and tungsten, and other high-density metals with stronger developing capacity.

It is obvious that the aforementioned embodiments of the present disclosure are only examples for clearly illustrating the present disclosure, rather than limiting the embodiments of the present disclosure. Various obvious changes, readjustments and replacements can be made by those skilled in the art without departing from the protection scope of the present disclosure. There is no need and no way to exhaust all of the embodiments. Any modification, equivalent replacement, and improvement, etc. made within the spirit and principle of the present disclosure shall be included in the protection scope of the present disclosure.

## Claims

1. A medical device, comprising a joint (1), a catheter (2) and a guiding wire (3), an end of the catheter (2) being connected to the joint (1), the guiding wire (3) being provided inside the catheter (2), wherein one end of the guiding wire (3) is connected to the joint (1), the other end of the guiding wire (3) extends out of the catheter (2), and an opening of the other end of the catheter (2) is sealed, the medical device further comprises:
an elastic portion (4) sleeved on the other end of the guiding wire (3); and
a protruding portion (5) sleeved on a periphery of the guiding wire (3), the protruding portion (5) is connected between the elastic portion (4) and the catheter (2), and a diameter of the protruding portion (5) is greater than diameters of the catheter (2) and the elastic portion (4).

2. The medical device according to the claim 1, wherein the protruding portion (5) comprises two protruding members (51), the protruding members (51) are shaped as a truncated cone, lower bottom surfaces of the two protruding members (51) are attached, and upper bottom surfaces of the two protruding members (51) are connected to the catheter (2) and the elastic portion (4), respectively.

3. The medical device according to claim 2, wherein the two protruding members (51) are symmetrically arranged.

4. The medical device according to claim 1, wherein a diameter of the guiding wire (3) gradually decreases from the joint (1) towards the elastic portion (4).

5. The medical device according to claim 1, wherein the diameter of the catheter (2) gradually decreases from the joint (1) towards the protruding portion (5).

6. The medical device according to claim 1, wherein a wall of the other end of the catheter (2) is provided with a plurality of liquid outlet holes (221), and the opening of the other end of the catheter (2) is sealed.

7. The medical device according to claim 6, wherein the catheter (2) comprises:
a first catheter portion (21) connected to the joint (1); and
a second catheter portion (22) connected between the first catheter portion (21) and the protruding portion (5), a hardness of the second catheter portion (22) is greater than a hardness of the first catheter portion (21), and the plurality of liquid outlet holes (221) are located on the second catheter portion (22).

8. The medical device according to claim 1, wherein the elastic portion (4) comprises at least one spring.

9. The medical device according to claim 8, wherein the elastic portion (4) comprises a second spring and a third spring, the second spring is sleeved on a periphery of the third spring, the end of the guiding wire (3) extending out of the catheter (2) is coplanar with an end of the second spring, and an end of the third spring is coplanar with the end of the second spring.

10. The medical device according to claim 9, wherein a fatigue strength of the second spring is greater than a fatigue strength of the third spring.

11. The medical device according to claim 9, wherein a developing capacity of the third spring is greater than a developing capacity of the second spring.

12. The medical device according to claim 1, wherein an end of the elastic portion (4) away from the catheter (2) is provided with a hemispherical terminal (41).
